# EUROPEAN PATENT SPECIFICATION

(11) **EP 2 916 772 B1**
(45) Date of publication and mention of the grant of the patent: **20.12.2017**
(21) Application number: 13792623.4
(22) Date of filing: 08.11.2013
(51) Int. Cl.: A61F 2/24

(54) **STENT SEALS AND METHODS FOR SEALING AN EXPANDABLE STENT**
STENTDICHTUNGEN UND VERFAHREN ZUR DICHTUNG EINES EXPANDIERBAREN STENTS
JOINTS D'ÉTANCHÉITÉ D'ENDOPROTHÈSE ET PROCÉDÉS DE SCELLEMENT ÉTANCHE D'UNE ENDOPROTHÈSE EXTENSIBLE

(30) Priority: 08.11.2012 US 201261724002 P
(43) Date of publication of application: 16.09.2015
(73) Proprietor: Symetis SA, 1024 Ecublens (CH)
(72) Inventor: DELALOYE, Stephane, 8180 Bülach (CH)
(74) Representative: Peterreins Schley
(86) International application number: PCT/EP2013/073318
(87) International publication number: WO 2014/072439

(56) References cited:
- EP-A1- 2 047 824
- WO-A1-2010/083558
- WO-A1-2011/057087
- WO-A1-2013/033791
- WO-A1-2013/134214
- US-A1- 2005 137 695

## Description

### Field of the Disclosure

The present disclosure relates to the field of stents implantable in the body. Embodiments have been devised to address problems encountered in the field of stent-valves, for example cardiac stent-valves (e.g., prosthetic heart valves). However, the concepts disclosed herein may have broader application to any stent or stented prosthesis where a seal is desired at an exterior surface of a stent.

### Background of the Disclosure

Transcatheter valve implantation (for example, transcatheter aortic valve implantation (TAVI)) is an evolving technology for replacement valve therapy that (i) avoids the trauma of conventional open-chest surgery, and (ii) avoids the need for heart and lung bypass. In such a technique, a stent-valve is compressed and loaded into a delivery catheter. The delivery catheter is introduced to the desired site of implantation (for example at the heart) via a percutaneous route or via minimally invasive surgery. The stent-valve is expanded into the implantation position from or by the delivery catheter, and the delivery catheter is then withdrawn.

Despite the successes of transcatheter stent-valves, technological challenges remain. One such challenge is preventing retrograde leakage of blood around the stent-valve (so called para-valve leakage). The above-noted stents form a friction fit with the native anatomy to anchor the stent-valve in position, and are round in cross-section. However the native anatomy in which the stent is implanted is often off-round and is different for each person. Moreover, heavy calcification of the native anatomy may obstruct full deployment of any stent and make the native anatomy even more irregular. Thus, it can sometimes be difficult to provide a perfectly sealing fit between the stent-valve and the surrounding anatomy. Para-valve leakage is believed to be one of the factors affecting the long-term efficacy of the prosthetic valve, and possibly the life expectancy of the patient. One explanation is that the heart may have to work harder to compensate for some blood leaking retrograde at the entrance or exit of the heart. Therefore, addressing para-valve leakage is a significant challenge.

It is known to incorporate an external skirt or cover as part of the stent-valve. For example, the skirt is made of compressible biocompatible material, such as pericardial tissue or PET. The thicker the material of the skirt, the more able the skirt is to occlude gaps and effect a seal. However, a disadvantage is that such skirts add to the bulk of the stent-valve. A thick skirt makes the stent-valve problematic to compress to a desirably small size for implantation.

US-A-2005/0137688 is understood to describe compliant sacs disposed around the exterior of a stent, that are said to provide a more efficient seal along an irregular interface. The sacs may be filled with an appropriate material, for example, water, blood, foam or a hydrogel. Different arrangements of sacs are proposed in principle, but this document neither describes any specific construction technique nor does it describe handling of the fill material.

US patent 5769882 is understood to describe an implantable expansible tubular vascular prosthesis carrying a form-in-place sealing layer for occluding at least a circumferential band at the interface between the prosthesis and the native tissue wall. In one example, the sealing layer comprises a hydrogel, arranged in a cuff comprising a permeable membrane.

EP 1262201 is understood to describe an implantable vascular device having an external seal structure comprising a swellablehydrodel. In use, the hydrogel absorbs a mass of liquid so as to assume, as a result of the absorption, a certain degree of mechanical consistency. An example hydrogel has a polyvinyl alcohol (PVA) base, in combination with a polysaccharide.

WO-A-2008/070442 is understood to describe prosthetic heart valves, both expanding and non-expanding types, each having an anchoring sleeve that changes shape when the valve is implanted, to prevent migration of the valve. The anchoring sleeve is at least partly made of a material that swells due to absorption of body fluids. In examples, the sleeve is made of an inner material that swells upon contact with body fluids, and enclosed by a cover.

US-A-2007/0060998 WO 2011/057087, WO 2013/134214, WO 2013/033791 and WO-A-2010/083558 are understood to describe delivery of a dispensable or releasable reactive sealing agent for endoluminal use around (at least substantially around) a prosthetic device within a body lumen. The reactive sealing agent is released or dispensed into a space between the prosthetic device and the lumen wall, in response to exertion of a dispensing pressure or by a configuration change causing the release. While different arrangements of dispensing capsules are proposed, reliable containment of the agent when the prosthesis is implanted at the heart likely are not ensured, especially in view of the constant movement and cyclic compression experienced by heart valves.

Accordingly, it would be desirable to address one or more of the above issues and/or provide a technique for mitigating para-valve (or para-stent) leakage without substantially affecting other desirable characteristics.

### Summary of the Invention

Aspects of the invention are defined in the claims.

The following disclosure presents a summary of the invention in order to provide a basic, non-limiting, understanding of some embodiments of the invention.

For example, in some embodiments of the present disclosure, a prosthesis comprising a stent and a seal for obstructing para-prosthesis leakage is provided. The prosthesis is a stent-valve (for example a cardiac stent-valve, such as an aortic stent-valve).
- The seal comprises a swellable material that swells in response to contact with blood.
- The seal may be captive within a hollow cuff that extends in a circumferential direction.
- The cuff may comprise flexible material. The cuff may comprise material that is elastically stretchable, and/or material that is substantially non-elastically-stretchable.
- The swellable material may occupy only a portion of the circumferential length of the cuff, for example, optionally not more than about 75%, optionally not more than about 60%, optionally not more than about 50%, optionally not more than about 40%, optionally not more than about 30%, optionally not more than about 25%, optionally not more than about 20%.
- The cuff may be transparent or translucent. The swellable material may have a distinctive color (at least when dry) enabling the position of the swellable material to be identified within the cuff. Such identification may aid a practitioner in deciding where optionally to puncture the cuff, if this technique is used, as described later.
- The cuff may have or comprise an integral tubular structure. As used herein, the term "integral tubular structure" may mean that the cuff (or in the case of a laminate, at least a structural substrate within the laminate) is produced as an original integral tube around an axis passing along a centerline of the tube. As used herein, references to the cuff having or comprising an integral tubular structure apply to at least a structural substrate of the laminate, whether or not mentioned explicitly, and whether or not the entire cuff may have such a structure. For example, integral tubular structures may be made by extrusion of material in tubular form, or by blow molding a preform to define a tubular form.

In some embodiments, an integral tubular structure contrasts from a tube that is non-integrally formed around an axis passing along a centerline of the tube. Non-integral forming may include, for example, wrapping a film or sheet around an axis and securing portions to the film or sheet to define a hollow envelope enclosed by the wrapping.

In some embodiments, using an integral tubular structure for the cuff may enable the cuff to achieve the otherwise conflicting requirements of desirably thin wall thickness, and good strength against bursting. Risk of bursting is often highest at join-lines of non-integral structures. Forming an integral tubular structure reduces the need for extensive join lines, in particular, a join line extending circumferentially around the prosthesis (in some embodiments, substantially around).

In some embodiments, in which the stent-valve is configured to be expanded to an operative configuration by expansion by an inflatable expansion balloon, providing a stent-valve with a seal cuff comprising an integral tubular structure may be highly advantageous in enabling the seal cuff to made desirably thin, yet have good strength and resistance to bursting should the seal be subject to the forces applied during the balloon-expansion, especially against the irregular or sharp contours of a calcified native anatomy

Although not immediately intuitive, some embodiments of the present disclosure provide a technique of post-implantation balloon-expansion of an implanted prosthesis stent-valve carrying a swellable seal. Providing a stent-valve with a seal cuff comprising an integral tubular structure may be highly advantageous in enabling the seal cuff to made desirably thin, yet include strength and resistance to bursting should the seal be subject to the high forces applied during post-implantation balloon-expansion, especially against the irregular or sharp contours of a calcified native anatomy. For example, such forces may be greater than normally experienced by the seal during initial implantation (whether by self-expansion of the stent-valve, or by manual manipulation, for example, initial balloon expansion). Additionally or alternatively, it may permit a second implantation procedure (for example, even many years into the future), which may itself involve a valvuloplasty procedure using an expansion balloon to prepare for implantation of a further prosthesis. The fact that the current stent-valve comprises a seal configured to withstand balloon expansion (e.g. valvuloplasty) forces without risk of bursting, may continue to provide the patient with the full range of options for future treatment (which might not be available to a patient who has been implanted with a different type of swelling seal not designed to withstand a future balloon expansion and/or valvuloplasty procedure).

Continuing from the list above, the seal may comprise one or any combination of two or more of the following features, as well as the above-noted features, which are all optional.
- Whether or not the cuff is formed as an integral tubular structure, the cuff may comprise a tubular extrusion or blow molded tubing.
- In addition to, or as an alternative to, the above, the cuff may be configured to be able to withstand post-implantation balloon expansion of the stent-valve against a calcified anatomy without substantial loss of structural integrity of the hollow cuff. This can provide similar advantages for permitting balloon expansion (e.g. post-implantation balloon expansion) and/or suitability for a future valvuloplasty procedure.
- The cuff may be formed by a method including providing an elongate hollow tubular member (optionally with an integral tubular structure), introducing the swellablematerial into the interior of the tubular member, and bending the elongate tubular member to form a substantially toroid shape.
- The opposite ends of the bent elongate tubular member may be secured together (for example, by fusion, welding, or adhesive) to define a closed-loop toroid form, whether or not the ends of the tube communicate openly with each other as a continuous open interior space.
- The hollow cuff may be formed from or using a tubular segment from an inflatable cardiacvalvulopasty balloon. Such balloon material already has desirable characteristics of being thin-walled yet strong to resist bursting when the balloon is inflated and bears directly against hard, irregular and sharp calcifications of a calcified vascular anatomy. The balloon material is also established as being bio-compatible and suitable for introduction into, and for direct contact with, the human vasculature.
- The hollow cuff may be liquid-tight, at least prior to use of the stent-valve.
- The hollow cuff may be of polymeric material and carry a diffusion barrier layer to obstruct diffusion of liquid through the cuff wall and into the space containing the swellable material.
- The hollow cuff may comprise a laminate of (1) plastics film and (2) a diffusion barrier layer to obstruct diffusion of liquids from outside the hollow cuff to the hollow interior. The diffusion barrier optionally is formed either on an interior face of the cuff (e.g. the hollow interior face), or as a non-surface layer of the laminate. Such positioning of the diffusion barrier layer may protect the integrity of the diffusion barrier layer during production and assembly of the stent-valve, enabling easier handling.
- The diffusion barrier material of either of the above may be of or comprise a metal or metal compound (e.g., an oxide).
- The metal or metal compound may be formed by plasma vapour deposition. The thickness of the layer may be optionally less than 100nm, optionally less than 50nm, optionally less than 10nm.
- The diffusion barrier layer may be configured to remain in position on the stent-valve when the valve is implanted.
- Additionally or alternatively, the cuff material is configured to be pierced in use, prior to introduction into the body of a patient, to create liquid-admitting punctures in the cuff material.
- The prosthesis may be provided as part of a kit including a piercing tool usable to pierce the cuff to form liquid-admitting punctures in the cuff. The piercing tool may comprise at least one pin or other sharp projection. The (or each) pin or protection may be dimensioned to permit puncturing of the cuff without damaging other operative portions of the prosthesis (for example, without damaging leaflets of a stent-valve).
- In some embodiments, the cuff may be pierced before, during, or after, loading of the stent-valve into a delivery catheter. In some examples, the delivery catheter includes a sheath within which the stent-valve is at least partly contained when loaded (or during loading). The sheath may include at least one (and optionally a plurality) of apertures aligned with the cuff, and through which the piercing tool may be introduced to pierce the cuff while in situ in the delivery catheter.
- In one example condition of a stent-valve prior to introduction of the stent-valve into the body of a patient, the stent-valve has a seal cuff containing a swellable material. The cuff is liquid impermeable except for at least one (and optionally a plurality) of liquid-admitting punctures made therein, for admitting liquid into the seal. Additionally or alternatively, the cuff is made of liquid-impermeable material, the cuff having one or more liquid admitting punctures made therein for admitting liquid into the seal.
- In one example condition of a stent-valve prior to introduction of the stent-valve into the body of a patient, the stent-valve according to some embodiments includes a seal cuff containing a swellable material. The swellable material is at least partly hydrated or wetted by liquid (e.g., prior to introduction of the stent-valve into the body). The cuff may be constrained against substantial expansion by being constrained within a sheath of a delivery apparatus. The hydrating or wetting liquid may, for example, be saline. Allowing the seal cuff to at least partly hydrate or become at least party wetted prior to introduction into the body may enable more efficient swelling of the material, and therefore of the cuff, when the stent-valve is implanted. It can avoid the need for the seal to have to become wetted by liquid only on implantation. For example, speed of wetting and/or swelling may be a consideration if the liquid-admitting apertures (e.g. punctures) in the cuff are relatively small and/or if a relatively "slow" hydrating/swelling material is used within the cuff.
- Additionally or alternatively to the above, in one example condition of a stent-valve prior to introduction of the stent-valve into the body of a patient, the stent-valve is loaded at least partly into a delivery catheter. The delivery catheter comprises a containment sheath encompassing at least a portion of the stent-valve at which the seal is located, the containment sheath being at least partly filled with liquid, and the swellable material being exposed to the liquid, the containment sheath obstructing expansion of the seal. The liquid may, for example, be saline.
- The seal may further comprise a skirt secured to the hollow cuff, for example, using an attachment that does not puncture the cuff. Example attachments may include one or more of: fusion; welding, adhesive. The skirt may itself be attached to the stent, for example, by sutures. The skirt may provide a means by which the seal is fixed to the stent. Such a technique can enable the seal to be secured fixed to the stent, without risk that the stent fixings may compromise the integrity of the cuff.
- The stent-valve (optionally all of the stent, valve-leaflets, and seal) may be compressible to a compressed configuration for delivery, and expandable to an operative configuration at implantation. In some embodiments, the stent is a self-expanding type that self-expands at least partly towards (and preferably self-expands entirely to) the operative configuration. Additionally or alternatively, the stent may be manually manipulable (e.g. plastically expandable) to the operative configuration, for example, using an expansion balloon or other expanding device or foreshortening device. The material of the stent, in either case, may for example be selected from one or more of: shape memory material; shape memory metal alloy; nitinol; steel, nickel-chromium (containing) alloy; chromium-cobalt (containing) alloy.

Further embodiments of the disclosure may relate to a method of production of a stent-valve, optionally as defined by any one or any combination of two or more of the foregoing aspects and features. The method may comprise one or any combination of two or more of the following steps and/or features, which are all optional.
- A seal of the stent-valve may be provided comprising a liquid-tight sealed cuff containing a swellable material.
- After assembling the components of the stent-valve (e.g. a stent, one or more prosthetic valve leaflets, and the seal), the stent-valve may be immersed into a liquid. For example, the liquid may be a sterilizing liquid and/or a preservative liquid for packaging the stent-valve ready for use.
- The liquid-tight sealed cuff may prevent the liquid from contaminating the swellable material of the seal prior to intended use.
- The liquid may be toxic to the human blood stream (for example, intended to be rinsed or otherwise cleaned off the stent-valve before the stent-valve is introduced into a patient's body).
- A tubular cuff of the seal (and/or a structural substrate of a laminate thereof) may be formed by an integral tubular forming technique. Example techniques may include tubular extrusion and/or blow molding.
- A tubular cuff of the seal (and/or a structural substrate of a laminate thereof) may be obtained from a segment of a valvuloplasty balloon.
- A tubular cuff may be provided by the steps including providing an elongate hollow tubular member (optionally with an integral tubular structure), introducing the swellable material into the interior of the tubular member, and bending the elongate tubular member to form a substantially toroid shape.
- The opposite ends of the bent elongate tubular member may be secured together (for example, by fusion, welding, or adhesive) to define a closed-loop toroid form. The opposite ends may be sealed closed to define a non-continuous interior of the cuff at the join in the toroid, or they may communicate with each other to define a continuous open interior across the join.
- A diffusion barrier layer may be formed on a tubular cuff of the seal, or on a material blank used to form the tubular cuff, or on other cover for a seal comprising swellable material.
- The diffusion barrier layer may be or comprise a metal or metal compound.
- The diffusion barrier layer may be formed by plasma vapour deposition.
- The method may include sterilizing a component used to form the liquid-tight sealed cuff containing swellable material, by irradiation.
- The method may include sterilizing the stent-valve, after assembly, by contacting the stent-valve with a sterilizing fluid, e.g. immersing the stent-valve in a sterilization liquid. The liquid-tight sealed cuff may prevent liquid contamination of the swellable material. The sterilization liquid may optionally comprise an aldehyde, optionally glutaraldehyde.
- The method may include storing the stent-valve, ready for use, in liquid preservative. The liquid-tight sealed cuff may prevent liquid contamination of the swellable material. The liquid preservative may optionally comprise an aldehyde, optionally glutaraldehyde.
- The method may include sterilizing a sealed cuff and swellable material therein, using a different sterilizing technique from the remainder the stent valve. For example, the cuff and the swellable material may be sterilized using radiation. The remainder of the stent-valve may be sterilized by contacting the stent-valve with a sterilizing liquid (or other sterilizing fluid).

Further embodiments of the present disclosure may relate to a method of using a stent-valve for implantation, the stent-valve optionally as defined and/or produced by any one or any combination of two or more of the foregoing aspects and features. The method of using may comprise one or any combination of two or more of the following steps and/or features, which are all optional:
- providing the stent-valve stored in a storage solution, the stent-valve including a seal comprising a material that swells when contacted by liquid and a cuff or cover protecting the seal from contact by the storage solution;
- rinsing the stent-valve to clean the stent-valve of the storage solution;
- after rinsing, piercing the cuff or cover at one or more positions to break the integrity of the cuff or cover, in order to allow blood to contact the swellable material upon implantation;
- after rinsing, compressing and/or loading the stent-valve into a delivery apparatus for introduction into the body;
- additionally or alternatively to step (c), after rinsing and while the stent-valve is outside a human body, exposing the swellable material to, and/or contacting the swellable material with, liquid to allow at least partial wetting or hydration of the swellable material; and
- feature (e) may be carried out before, or during, or after step (d). For example, the liquid may be liquid within which the stent-valve is at least partly immersed (i) during compressing and/or loading, or (ii) within the delivery catheter.

In a closely related aspect, a description of the use of the stent-valve is given, the stent-valve optionally as defined and/or produced by any one or any combination of two or more of the foregoing aspects and features. The method of using may comprise one or any combination of two or more of the following steps and/or features, which are all optional:
- providing a stent-valve that is compressible to a compressed configuration for delivery, and expandable to an operative configuration for implantation, the stent-valve comprising a stent, a plurality of leaflets defining a prosthetic valve, and a seal for sealing against surrounding tissue, the seal comprising a swellable material that swells when contacted by blood;
- introducing the stent-valve into the body in its compressed configuration using a delivery device, and advancing the stent-valve to a desired implantation site;
- causing the stent-valve to expand at the implantation site, from the compressed configuration to its operative configuration;
- observing one or more characteristics of the operative stent-valve; and
- in dependence on the result of the observation at step (d), performing post-implantation balloon expansion of the stent-valve.

Features and advantages of some of the embodiments of the disclosure, include:
- facilitating a seal construction that is able to swell to automatically seal gaps between the stent-valve and the surrounding tissue, even in the case of an irregular anatomy;
- facilitating safe post-dilation of the stent-valve as desired, without significant risk of seal rupture;
- facilitating long storage times of a stent-valve without risk of contaminating the swellable material of the seal by toxic storage solution;
- facilitating thorough sterilization of a stent-valve without contaminating or otherwise compromising the swellable material of a seal;
- facilitating simple yet effective activation of the swellable material of a seal without having to separate components;
- facilitating early partial hydration or wetting of a swellable seal before implantation, to reduce the burden of seal to access liquid only at the instant of deployment at the implantation site;
- avoiding the need for any rupture of a capsule membrane during the implantation process, by facilitating exposure of a swellable seal material to liquid prior to introduction into the body, and carrying out such exposure while the seal is constrained against expansion.

In a related independent aspect, the invention provides a stent-valve delivery system comprising a delivery catheter loaded with a stent-valve. The stent-valve may comprise a seal comprising swellable material that swells when contacted by liquid.The delivery catheter may comprise a containment sheath encompassing at least a portion of the stent-valve at which the seal is located. The containment sheath may be at least partly filled with liquid.The swellable material may be exposed at least partly to the liquid. The containment sheath may obstruct expansion and/or outward swelling of the seal.

In a related independent aspect, the invention provides a stent-valve delivery system outside the body of a patient and comprising a delivery catheter loaded with a stent-valve. The stent-valve may comprise a seal comprising swellable material that swells when contacted by liquid.The swellable material may be in an at least partly hydrated condition by contact with liquid.The delivery catheter may comprising a containment sheath encompassing at least a portion of the stent-valve at which the seal is located. The containment sheath may obstruct outward swelling of the at least partly hydrated swellable material.

In a related independent aspect, the invention provides a stent-valve delivery system comprising a stent-valve and a delivery catheter. The stent-valve may comprise a seal comprising swellable material that swells when contacted by liquid.The delivery catheter may comprise a containment sheath for encompassing at least a portion of the stent-valve at which the seal is located at least when the sheath is in a closed position, and a port for introduction of liquid into the containment sheath. In use, once the stent-valve is loaded to the delivery catheter, the swellable material may be exposed to liquid introduced through the port into the containment sheath. Outward swelling of the swellable material may be obstructed by the containment sheath.

In a related independent aspect, the invention provides a method of preparing a stent-valve for implantation, comprising the steps of:
(a) providing a stent-valve stored in a storage solution, the stent-valve comprising a seal comprising a material that swells when contacted by liquid, and a cuff protecting the seal from contact by the storage solution;
(b) rinsing the stent valve to clean the stent-valve of the storage solution;
(c) after at least step (b) and while the stent-valve is outside a human body, exposing the swellable material to, and/or contacting the swellable material with, liquid to allow at least partial wetting or hydration of the swellable material by the liquid;
(d) after at least step (b) and while the stent-valve is outside of a human body, compressing and/or loading the stent-valve into a delivery catheter for introduction into a patient's body.

In a related independent aspect, the invention provides a method of production of a stent-valve, comprising:
(a) providing a stent that is that is compressible to a compressed configuration for delivery, and expandable to an operative configuration for implantation;
(b) providing a prosthetic valve component;
(c) providing a seal for sealing against surrounding tissue, the seal comprising a liquid-tight sealed cuff containing a swellable absorbent material configured to swell when contacted by liquid, to distend the external seal;
(d) assembling the prosthetic valve component and the seal to the stent to form the stent-valve;
(e) contacting the stent-valve with a fluid that is toxic to the human bloodstream, and wherein the liquid-tight sealed cuff prevents the fluid from toxically contaminating the swellable absorbent material of the seal.

In a related independent aspect, the invention provides a method of production of a stent-valve, comprising:
(a) providing a stent that is that is compressible to a compressed configuration for delivery, and expandable to an operative configuration for implantation;
(b) providing a prosthetic valve component;
(c) providing a seal for sealing against surrounding tissue, the seal comprising a liquid-tight sealed cuff containing a swellable material configured to swell when contacted by liquid, to distend the external seal, the cuff having a sterile interior;
(d) assembling the prosthetic valve component and the seal to the stent to form the stent-valve;
(e) sterilizing the assembled stent-valve by contacting the stent-valve with a sterilizing fluid for sterilizing portions of the stent-valve contacted by the liquid,
and wherein the liquid-tight sealed cuff prevents the sterilizing fluid from contaminating the swellable absorbent material of the seal.

In a related independent aspect, the invention provides a method of production of a stent-valve, comprising:
(a) providing a stent that is that is compressible to a compressed configuration for delivery, and expandable to an operative configuration for implantation;
(b) providing a prosthetic valve component;
(c) providing a seal for sealing against surrounding tissue, the seal comprising a liquid-tight sealed cuff containing a swellable material configured to swell when contacted by liquid, to distend the external seal, the cuff being made of or comprising at least partly a flexible laminate of (i) plastics film and (ii) a diffusion barrier layer to obstruct diffusion of liquids from outside the hollow cuff to the hollow interior, the diffusion barrier layer comprising metal or a metal compound;
(d) assembling the prosthetic valve component and the seal to the stent to form the stent-valve.

In a related independent aspect, the invention provides a method of producing an seal assembly that is suitable for use as a seal of a stent-valve for sealing against surrounding tissue at a site of implantation, the method comprising:
(a) providing a flexible elongate hollow tubular member, the tubular member comprising an integral tubular structure;
(b) introducing into the interior of the hollow tubular member, a swellable material that swells when contacted by liquid;
(c) bending the elongate hollow tubular member to form a substantially toroid shape.

In a related independent aspect, the invention provides a method of production of a stent-valve that is compressible to a compressed configuration for delivery, and expandable to an operative configuration for implantation, the stent-valve comprising a stent, a plurality of leaflets defining a prosthetic valve, and a seal for sealing against surrounding tissue, the seal comprising a liquid-tight sealed cuff containing a swellable material configured to swell when contacted by blood to distend the external seal, the method comprising sterilization steps, including in any order:
exposing, to radiation, at least component that forms the liquid-tight sealed cuff, to sterilize the interior of the cuff and the swellable material therewithin; and
contacting the stent-valve with a sterilizing liquid to sterilize portions of the stent-valve contactable by the fluid, the liquid-tight sealed cuff preventing the liquid from contacting the swellable material.

Not part of the invention, but of background interest is a stent-valve for transcatheter implantation to replace a cardiac valve, the stent-valve being compressible to a compressed configuration for delivery, and expandable to an operative state for implantation. The stent-valve may comprise a stent, a plurality of leaflets defining a prosthetic valve, and a seal for sealing against surrounding tissue.The seal may comprise a hollow cuff arranged to extend in a circumferential direction substantially around the stent and containing swellable material that swells when contacted by blood to distend the hollow cuff. The hollow cuff may comprise an integral tubular structure.

In a related independent aspect, also not falling under the scope of the claims is a stent-valve for transcatheter implantation to replace a cardiac valve, the stent valve being compressible to a compressed configuration for delivery, and expandable to an operative state for implantation. The stent-valve may comprisw a stent, a plurality of leaflets defining a prosthetic valve, and a seal for sealing against surrounding tissue. The seal may comprisea hollow cuff arranged to extend in a circumferential direction substantially around the stent and containing swellable material that swells when contacted by blood to distend the hollow cuff.The hollow cuff may comprise a tubular extrusion or blow molded tubing.

A related device, also not covered by the scope of the claims provides a stent-valve for transcatheter implantation to replace a cardiac valve, the stent valve being compressible to a compressed configuration for delivery, and expandable to an operative state for implantation. The stent-valve may comprise a stent, a plurality of leaflets defining a prosthetic valve, and a seal for sealing against surrounding tissue.The (e.g. external) seal may comprisea hollow cuff arranged to extend in a circumferential direction substantially around the stent and containing swellable material that swells when contacted by blood to distend the hollow cuff. The hollow cuff may comprise a laminate of (i) plastics film and (ii) a diffusion barrier layer to obstruct diffusion of liquids from outside the hollow cuff to the hollow interior. The diffusion barrier layer may comprise a layer comprising metal or a metal compound.

Another example of the related art describes a stent-valve for transcatheter implantation to replace a cardiac valve, the stent valve being compressible to a compressed configuration for delivery, and expandable to an operative state for implantation. The stent-valve may comprise a stent, a plurality of leaflets defining a prosthetic valve, and a seal for sealing against surrounding tissue.The (e.g. external) seal may comprisea hollow cuff arranged to extend in a circumferential direction substantially around the stent and containing swellable material that swells when contacted by blood to distend the hollow cuff. The hollow cuff may be configured to withstand post-implantation balloon expansion of the stent-valve against a calcified anatomy without substantial loss of structural integrity of the hollow cuff.

Yet another example of a related device provides a stent-valve for transcatheter implantation to replace a cardiac valve, the stent valve being compressible to a compressed configuration for delivery, and expandable to an operative state for implantation. The stent-valve may comprise a stent, a plurality of leaflets defining a prosthetic valve, and a seal for sealing against surrounding tissue. The (e.g. external) seal may comprisea hollow cuff arranged to extend in a circumferential direction substantially around the stent and containing swellable material that swells when contacted by blood to distend the hollow cuff. The hollow cuff may comprisr a film made of liquid-impermeable material. The cuff may have one or more liquid admitting punctures made therein, prior to introduction of the stent-valve into the body of a patient, for admitting liquid into the seal.

In a related aspect, also not part of the invention there is a kit comprising a stent-valve and a piercing tool. The stent-valve may be for transcatheter implantation to replace a cardiac valve, the stent valve being compressible to a compressed configuration for delivery, and expandable to an operative state for implantation. The stent-valve may comprise a stent, a plurality of leaflets defining a prosthetic valve, and a seal for sealing against surrounding tissue.The seal may comprise a liquid-tight hollow cuff arranged to extend in a circumferential direction substantially around the stent and containing swellable material that swells when contacted by blood to distend the hollow cuff. The piercing tool may be manually usable to pierce the cuff to form liquid admitting punctures in the cuff.

In a related independent aspect, the example, not part of the invention, provides a method of preparing a stent-valve for implantation, comprising the steps of:
(a) providing a stent-valve stored in a storage solution, the stent-valve comprising a seal comprising a material that swells when contacted by liquid, and a cuff protecting the seal from contact by the storage solution;
(b) rinsing the stent valve to clean the stent-valve of the storage solution;
(c) after rinsing, using a piercing tool to piercing the cuff at one or more positions to define one or more liquid-admitting punctures for admitting liquid into the cuff to communicate with the swellable material;
(d) after rinsing, compressing and/or loading the stent-valve into a delivery catheter for introduction into a patient's body.

An example of the use of the device comprises:
(a) providing a stent-valve that is compressible to a compressed configuration for delivery, and expandable to an operative configuration for implantation, the stent-valve comprising a stent, a plurality of leaflets defining a prosthetic valve, and a seal for sealing against surrounding tissue, the seal comprising a swellable material that swells when contacted by blood;
(b) introducing the stent-valve into the body in its compressed configuration using a delivery device, and advancing the stent-valve to a desired implantation site;
(c) causing the stent-valve to expand at the implantation site, from the compressed configuration to its operative configuration;
(d) observing one or more characteristics of the operative stent-valve; and
(e) in dependence on the result of the observation at step (d), performing post-implantation balloon expansion of the stent-valve.

Additional and/or independent embodiments and features of the disclosure are included in the claims.

Although certain features and aspects of the invention are highlighted in the foregoing summary and in the appended claims, protection is claimed for any novel concept described herein and/or illustrated in the drawings, as defined in the scope of the claims.

### Description of the Drawings

Non-limiting embodiments of the invention are now described with reference to the accompanying drawings, in which :
Fig. 1 is a schematic drawing illustrating a stent-valve 10 with which some embodiments of the present disclosure are suitable to be used. The figure is broken along a centre-line of the stent-valve. The stent-structure is shown to the right, and a profile showing the positions of the valve, skirt and seal is shown to the left.
Fig. 2 is an enlarged schematic section showing the seal of Fig. 1 in isolation.
Fig. 3a is a schematic perspective view of an elongate tubular member for use in the production of a seal according to some embodiments of the disclosure.
Fig. 3b is a schematic section illustrating obtaining the tubular member from a valvulopasty balloon according to some embodiments of the disclosure.
Fig. 3c is a schematic partial perspective view of a sub-assembly including tubing and outer skirt material according to some embodiments of the disclosure.
Fig. 3d is a schematic section illustrating an example of forming the sub-assembly of Fig. 3c, according to some embodiments of the disclosure.
Fig. 3e is a schematic view illustrating insertion of swellable material into the sub-assembly of Fig. 3c.
Fig. 3f is a schematic side view illustrating assembly of the sub-assembly to the stent of Fig. 1.
Fig. 3g is a schematic side view illustrating formation of a conical tubular sub-assembly for assembly to the stent of Fig. 1.
Fig. 4 is a schematic section illustrating a seal cuff provided with a diffusion barrier layer according to some embodiments of the disclosure.
Fig. 5 is a schematic flow diagram illustrating steps of a method for producing a stent-valve according to some embodiments of the disclosure.
Fig. 6 is a schematic section illustrating steps of a method of preparing a stent-valve for implantation according to some embodiments of the disclosure.
Fig. 7 is a schematic side view of a piercing tool for piercing a seal cuff of a stent-valve according to some embodiments of the disclosure.
Fig. 8 is a schematic section of a first example of delivery catheter containing a stent-valve loaded therein according to some embodiments of the disclosure.
Fig. 9 is a schematic section of a second example of delivery catheter containing a stent-valve loaded therein according to some embodiments of the disclosure.
Fig. 10 is a schematic flow diagram illustrating steps of a method of implanting a stent-valve according to some embodiments of the disclosure.

### Description of Preferred Embodiments

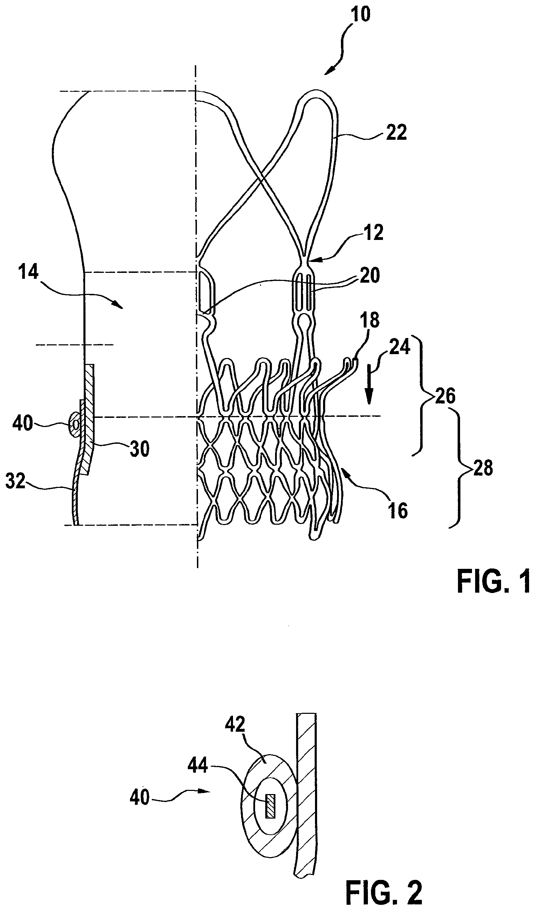

Referring to Fig. 1, a stented prosthesis according to some embodiments is illustrated in the form of a stent-valve 10. The stent-valve may include a seal 40 (described further below) for sealing against surrounding tissue when the stent-valve 10 is implanted. The stent-valve 10 may be cardiac stent-valve, for example, an aortic stent-valve, a mitral stent-valve, a pulmonary stent-valve or a tricuspid stent-valve, for implantation at the respective valve position in a human heart.

The stent-valve 10 may optionally comprise biological tissue (for example, pericardium (such as porcine pericardium and/or bovine pericardium) and/or natural cardiac valve leaflets (for example, natural porcine cardiac valve leaflets, optionally attached to a portion of natural cardiac wall tissue). The biological tissue may be fixed, for example, using glutaraldehyde.

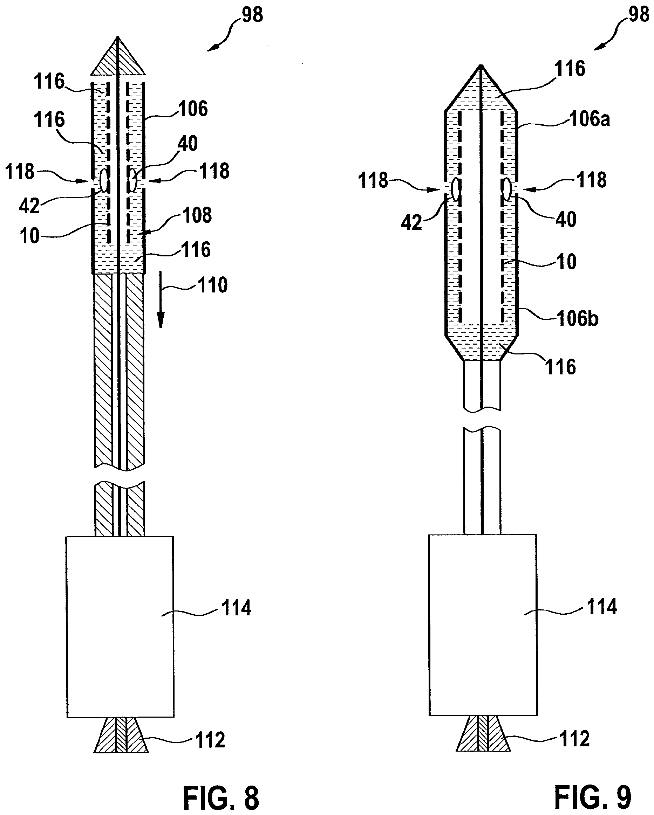

The stent-valve 10 may be compressible to a radially compressed condition (Fig. 8) for delivery using a delivery catheter, and be expandable to an operative or expanded condition (as shown) at implantation. The stent-valve 10 may comprise a stent 12 carrying a plurality of leaflets defining a valve 14 (the position of which is depicted schematically by the bounding phantom lines). Various geometries of stent 12 may be used. In some embodiments, the stent 10 may include one of more of: a lower tubular or crown portion 16, an upper crown portion 18, a plurality of upstanding commissural supports 20, and a plurality of stabilization arches 22. In use, the lower portion 16 of the stent 12 may be configured to be deployed after the other regions of the stent 12. For example, the arches 22, the supports 20 and the upper crown 18 may be deployed at least partly before the lower portion 16 (in that order, or in reverse order, or in a different order). At the very least, once the upper crown 18 has been at least partly deployed, the stent 12 may be urged and/or displaced in the direction of arrow 24 to seat the upper crown 18 against native leaflets at the implantation site. Deploying the lower portion 16 last fixes the stent 12 in its final position.

The lower portion 16, and optionally a portion of the upper crown 18, may be formed by a lattice structure of the stent. The lattice structure may define cells or apertures, for example, generally diamond-shaped apertures.

The native leaflets may generally overlap a portion 26 of the stent. The native valve annulus may overlap a portion 28 of the stent.

Optionally, the stent-valve 10 may further include an inner skirt 30 communicating with the leaflets 14 and carried on an interior of the stent 12. Additionally or alternatively, the stent-valve 10 may further comprise an outer skirt 32 carried on an exterior of the stent 12. When both skirts are provided, the skirts may partially overlap. The skirts may be offset such that one skirt (e.g. the outer skirt 32) extends further towards a lower extremity of the stent 12 than the other (e.g. inner skirt 30). Additionally or alternatively, one skirt (e.g. the inner skirt 30) extends further towards an upper extremity of the stent 12 than the other (e.g. outer skirt 32). The skirts may be of any suitable flexible and/or compliant material, for example, fabric (e.g. of PET) or of biological tissue (e.g. of pericardium). The inner and outer skirts may be secured to each other (for example, by sutures, welding or adhesive) along a generally continuous (e.g. circumferential) line or band of attachment, to obstruct leakage between the skirts. The attachment (e.g. sutures) may pass through and/or around the stent structure.

The valve 14 may comprise biological tissue, for example, pericardium (such as porcine pericardium or bovine pericardium) or natural cardiac valve leaflets (for example, natural porcine cardiac valve leaflets, optionally attached to a portion of natural cardiac wall tissue). Other biological or non-biological material could also be used for the valve 14, as desired.

The stent 12 may optionally be of a self-expanding type that is compressible to the compressed configuration for loading into a delivery catheter 98 (Fig. 8) having a sheath 106 for constraining the stent 12 in the compressed configuration for delivery to the site of implantation. In use, by removal of the constraining effect of the sheath, the stent 12 self-expands to or towards the operative configuration. A self-expanding stent may, for example, be of shape-memory material, for example, shape-memory metal alloy, for example, nitinol. Alternatively, the stent 12 may be configured to be expanded by application of a foreshortening force from the delivery catheter and/or by application of expanding force from the delivery catheter, such as by using an expansion balloon.

The stent-valve 10 may further comprise the seal 40 for sealing against surrounding native tissue when the stent-valve 10 is implanted. The seal 40 may be arranged at any suitable position on the stent 12. In some embodiments, the seal 40 may be arranged between the upper crown portion 18 and the lower crown or tubular potion 16. In some embodiments, the seal 40 may be positioned optionally closer to the upper crown portion 18, alternatively optionally closer to the lower crown or tubular portion 16, alternatively optionally midway between the extremities of the two crown portions 16 and 18, alternatively optionally at a waist or trunk section between the two crown portions 16 and 18. In some embodiments, the seal 40 is carried on the exterior of the stent 12.

Referring to Fig. 2, the seal 40 may comprise a hollow cuff 42 arranged to extend substantially in a circumferential direction around the stent 12, and containing swellable material 44 that swells when contacted by blood to distend the hollow cuff 42. The swellable material 44 may expand by absorbing blood or other liquids that contact the material 44. Such a seal 40 may initially be very compact in form, yet may expand significantly when contacted by blood, to fill gaps between the stent-valve 10 and any irregularities in the surrounding tissue. Examples of suitable swellable (e.g. absorptive) material 44 may be any of the hydrogels referred to in the aforementioned patents and applications: US 5769882, EP 1262201, WO-A-2008/070442, US 2007/0060998, WO-A-2010/083558. The cuff 42 may comprise flexible material. The cuff 42 may comprise material that is elastically stretchable, and/or material that is substantially nonelastically-stretching.

In some embodiments, the hollow cuff 42 has or comprises an integral tubular structure. An integral tubular structure may mean that the cuff 42 is produced as or comprises an original integral tube around an axis passing along a centerline of the tube; in the case of the cuff 42 having a laminate structure, at least a structural substrate (e.g. substrate layer) within the laminate may be produced as an integral tube around an axis passing along a centerline of the tube. As used herein, references to the cuff 42 having or comprising an integral tubular structure also apply to at least a structural substrate of the laminate, whether or not mentioned explicitly, and whether or not the entire cuff 42 may have such a structure. For example, integral tubular structures may be made by extrusion of the cuff 42 material in tubular form, or by blow molding a preform to define a tubular form. Using an integral structure for the cuff 42 may enable the cuff 42 to achieve the otherwise conflicting requirements of desirably thin wall thickness, and good strength against bursting. Risk of bursting is often highest at join-lines of non-integral structures. Forming an integral tubular structure reduces the need for extensive join lines, in particular, a join line extending circumferentially around (and in some embodiments, substantially around) the prosthesis.

As illustrated later below, in some embodiments, an implantation method may include a step of (e.g., post-implantation) balloon-expansion of an implanted prosthesis stent-valve 10 carrying a seal 40. Providing the stent-valve 10 with a seal cuff 42 having an integral tubular structure may be highly advantageous in enabling the seal cuff 42 to made desirably thin, yet have good strength and resistance to bursting should the seal be subject to the high forces applied during (e.g. post-implantation) balloon-expansion, especially against the irregular or sharp contours of a calcified native anatomy.

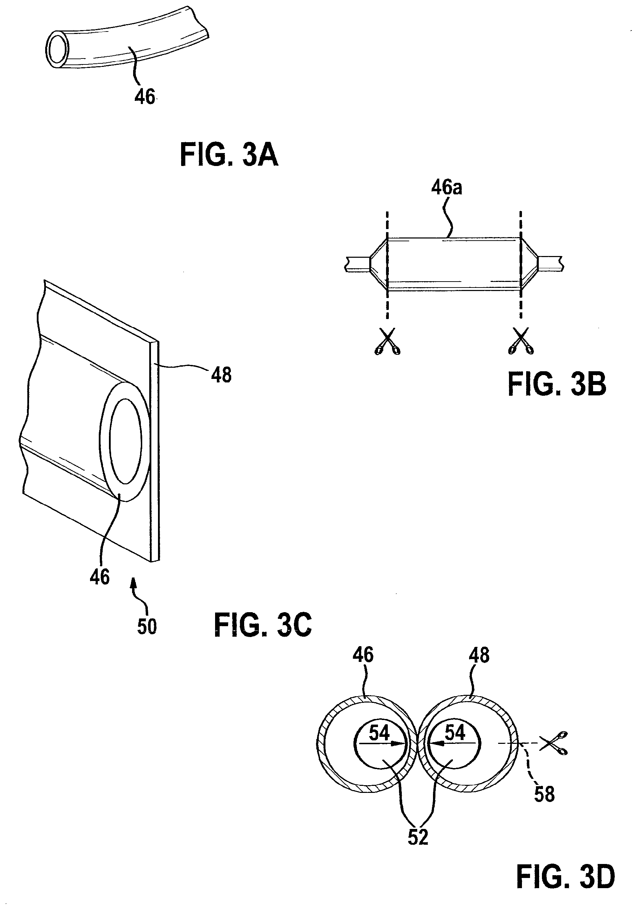

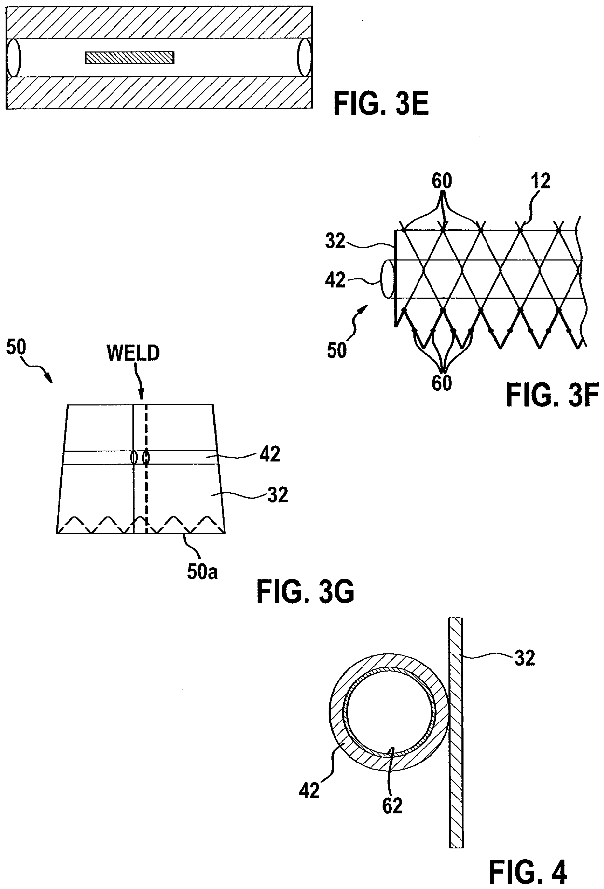

Referring to Fig. 3, whether or not of an integral tubular structure, the material for the cuff 42 may initially be provided in elongate tubular form 46 (Fig. 3a), for example, as an elongate integral tube. In some embodiments, (whether or not of an integral tubular structure) such an elongate tube 46 may be obtained from a balloon section of an inflatable cardiac valvuloplasty balloon 48 (Fig. 3b), for example, by cutting the balloon 46a near its ends, to extract an elongate tubular segment as the tube 46. Such balloon material already has desirable characteristics of being thin-walled yet strong to resist bursting when the balloon is inflated and bears directly against hard, irregular and sharp calcifications of a calcified vascular anatomy. The balloon material is also established as being bio-compatible and suitable for introduction into, and for direct contact with, the human vasculature.

Whether or not obtained from a cardiac valvuloplasty balloon, and whether or not having an integral tubular structure, example materials for the cuff 42, or tube 46, may include one or more of: polyamide (PA), polyimide (PI), polyetheretherketone (PEEK), polyester (PE), for example, polyethylene terephthalate (PET).

Referring to Fig. 3c, the elongate tube 46 may be attached to material 48, such as a material blank, for forming the outer skirt 32. The attachment of the tube 46 to the blank 48 is preferably by an attachment that does not puncture the elongate tube 46 for the cuff 42. The tubular integrity of the tube 46 may be preserved. The attachment may, for example, be by fusion, or welding, or adhesive. In some embodiments, the blank 48 may be of the same material as the tube 46, to facilitate attachment, for example, by fusion. Creation of a sub-assembly 50 comprising both the seal cuff 42 and the material 48 can facilitate easier handling during manufacture and production of the stent-valve 10.

Various techniques are possible. Purely by way of example, the material blank 48 may also be obtained from a section of a cardiac valvuloplasty balloon. Referring to Fig. 3d, the blank 48 may be manipulated while in tubular form. For example, mandrels 52 may be inserted into both the elongate tube 46 and the tubular blank 48. By a combination of heat and pressure (indicated by arrows 54), the tubes 46 and 48 may be fused together along an elongate line of attachment 56. Thereafter, the mandrels 52 are withdrawn, and the tubular blank 48 may be cut along a line 58 to define a planar section of material for the outer skirt 32.

Referring to Fig. 3e, the swellable material 44 may be placed into the interior of the elongate tube 46. The swellable material 44 may be substantially smaller (e.g., shorter) than the tube 46, but be able to swell significantly upon contact with blood, to distend the cuff 42 substantially around its periphery. The swellable material 44 may occupy only a portion of the circumferential length of the cuff, for example, optionally not more than about 75%, optionally not more than about 60%, optionally not more than about 50%, optionally not more than about 40%, optionally not more than about 30%, optionally not more than about 25%, optionally not more than about 20%. Optionally, the ends of the elongate tube 46 are each sealed to close the interior space of the tube 46 with the swellable material 44 captive therewithin. The ends may, for example, be sealed closed by welding, fusion, or adhesive.

Referring to Fig. 3f, the sub-assembly 50 may be bent into a tubular form, and attached to the stent 12. In some embodiments, the sub-assembly 50 is attached to the stent in sheet form, by wrapping the sub-assembly 50 around the stent 12. Alternatively, (Fig. 3g), the sub-assembly may be first secured in a tubular form, and the tubular form attached to the stent 12. For example, the ends of the sub-assembly may be partly overlapped and welded together, to define a lapped join. The weld may seal closed the ends of the cuff 42 (tubing 46). The weld may be clear of the swellable material within the cuff 42. The tubular sub-assembly 50 may have a conical shape to match the contour of the lower portion of the stent 12. The tubular assembly 50 may have a zig-zag edge 50a to match the peripheral edge at one end (e.g. inlet end) of the stent. For example, the zig-zag edge 50a may be cut and/or trimmed after assembly to the stent 12.

In either case, the sub-assembly 50 may be secured to the stent 12 by sutures 60. Optionally, the sutures 60 pass only through the material of the outer skirt 32, and do not penetrate the material of the cuff 42. The outer skirt 32 may act as the means for securing the cuff 42 to the stent 12 without compromising the tubular integrity of the cuff 42.

As can be seen in Fig. 3f, the elongate tube 46 is bent into a toroid shape around, or to match, the stent 12. The toroid shape may be a closed-loop toroid. Alternatively, the toroid shape may be partial loop, a split-loop, or a helical shape, for example. In some embodiments, the ends of the tube 46 are not sealed independently, but are sealed together to communicate with each other to define a circumferentially continuous hollow space across the join. However, in other embodiments, the ends of the tube 46 may be sealed closed to define a non-continuous interior across the join.

In some embodiments, the seal 40 and/or the cuff 42 may be directly (or indirectly) attached to the inner skirt 30, for example, by welding or adhesive or sutures. The attachment may be along a generally continuous (e.g. circumferential) line or band of attachment, to obstruct leakage between the seal 40 and the inner skirt 30. The attachment (e.g. sutures) may pass through or around the stent structure.

Referring to Fig. 4, the cuff 42 may carry or comprise a diffusion barrier layer 62. For example, the cuff material may comprise a laminate of (i) plastics film 64, and (ii) the diffusion barrier layer 62. The diffusion barrier layer 62 may serve to prevent diffusion of liquid, or other fluid, through the cuff wall material. As explained later below, the stent-valve 10 may be immersed in liquid or other fluid during manufacture (e.g. during sterilization) and/or during storage when packaged ready for use. The diffusion barrier layer 62 can substantially prevent any trace of liquid diffusing through the cuff wall, even though the plastics film 64 may be very thin.

In some embodiments, the diffusion barrier layer 62 is a metal or metal-compound. The diffusion barrier layer 62 may, for example, be deposited by plasma vapour deposition. The diffusion barrier layer 62 may have a thickness of less than 100 nm, optionally less than 50nm, optionally less than 10nm. The thickness of the diffusion barrier layer 62 may be exaggerated in Fug. 4. The diffusion barrier layer 62 may optionally be provided in a non-exterior-surface portion of the cuff wall. For example, the diffusion barrier layer 62 may be provided on an interior face of the cuff 42 (as shown in Fig. 4), or it may be provided as a non-surface portion of the laminate. Avoiding placing the diffusion barrier layer 62 on the exterior face of the cuff 42 may reduce the risk of damage to the integrity of the diffusion barrier layer 62, for example, during subsequent handling and production of the stent-valve.

When the diffusion barrier layer 62 is formed on a cuff 42 that has an integral tubular structure, plasma vapour deposition may, for example, be used to deposit the diffusion barrier layer in the hollow space of the cuff 42, on the interior face of the cuff 42. The diffusion barrier layer 62 may be deposited after the attachment of the cuff 42 (or the tube 46) to the material 48 for the outer skirt 32, to avoid risk of damage to the diffusion barrier layer during attachment of the cuff 42 or tube 46 to the material 48.

Alternatively, the exterior face of the cuff 42 or tube 46 may be coated with the diffusion barrier layer material, and a further protective coating (not shown) applied over the exposed face of the diffusion barrier layer, to complete the laminate.

In either case, the tube 46 may act as a structural substrate of the resulting laminate, providing the integral tubular structure of the cuff 42. Also, in either case, the diffusion barrier layer 62 may be an integral part of the stent-valve 10 that remains in place and is not removed at implantation.

Alternatively, the diffusion barrier layer may be provided on a removable portion of the cuff that is removed in order to expose the swellable material to liquid. The removable portion may, for example, be a liquid-tight cover.

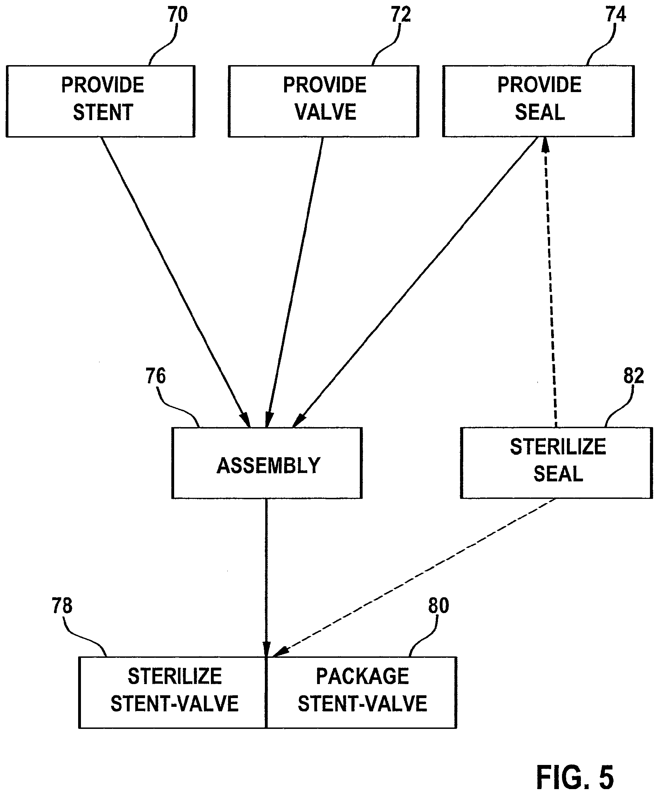

Referring to Fig. 5, a method of production of the stent-valve 10 may generally comprise one or more of the steps of:
Step 70: providing the stent 12;
Step 72: providing a prosthetic valve 14 (optionally attached to the inner skirt 30);
Step 74: providing the seal 40 (for example, the sub-assembly 50 including the cuff 42 containing the swellable material, and the material 48 for the outer skirt 32);
Step 76: assembling the valve 14 and the seal 40 to the stent 12, for example, using sutures to secure the valve 14 within the stent, and to secure the sub-assembly around an exterior portion of the stent 12:
Step 78: sterilizing the assembled stent-valve 10;
Step 80: placing the assembled stent-valve 10 into packaging for storage; and

Optionally step 82: sterilizing the seal 40 using a sterilization process different from step 78.

The step 78 of sterilizing the assembled stent-valve 10 may be performed by contacting the stent-valve 10 with a sterilization fluid, for sterilizing portions of the stent-valve contacted by the fluid. The fluid may, for example, be a liquid. Alternatively, the fluid may be a gas, or a liquid/gas combination. The sterilization fluid may be, or comprise a component, toxic to the human blood-stream. For example, the fluid may be intended to be rinsed or otherwise cleaned from the stent-valve prior to implantation. An example sterilization liquid comprises an aldehyde, for example, glutaraldehyde. The liquid may be an aqueous solution. Step 78 may optionally comprise heating the sterilization liquid to above room temperature, optionally above body temperature, optionally at least about 40°C, optionally at least about 50°C. Heating the sterilization liquid may enhance efficacy and/or speed of sterilization.

During step 78, the cuff 42 prevents the sterilizing fluid from contaminating the swellable material 44. As explained previously, the swellable material 44 may swell as a result of absorption of liquid. Toxic contamination of the swellable material 44 may make it difficult or impossible to remove the toxic liquid if chemically absorbed by the swellable material 44. Toxic contamination of the swellable material 44 may render the stent-valve less appropriate for implantation, and in some cases unimplantable. The cuff 42 may prevent such contamination (for example, even if a sterilization liquid is heated). If used, the diffusion barrier layer 62 may further enhance the protective properties of the cuff 42 in preventing any liquid from diffusing through the cuff into the space used for the swellable material.

Steps 78 and 80 may be carried out in either order, or at least partly at the same time. For example, in some embodiments, at step 80, the stent-valve 10 may be placed into its final packaging and immersed in liquid. The stent-valve may be sterilized in its final packaging, using the same liquid. Such a technique may be referred to as "terminal sterilization". In other embodiments, the stent-valve 10 may be sterilized by immersion in a first liquid (step 78), and subsequently transferred to a second liquid or storage liquid (step 80). The storage liquid may be similar to the sterilization liquid, and may be or comprise a component that is toxic to the human blood stream. In such case, provision of the cuff 42 (and optionally the diffusion barrier layer 62) protects the swellable material against toxic contamination. The stent-valve 10 may be stored in the storage liquid for an extended period of time. The cuff 42 may be configured to resist penetration and/or diffusion of the storage liquid to the interior space of the cuff, for a period of at least 1 month, optionally at least 6 months, optionally at least 1 year.

Step 82 may be an optional separate step of sterilizing the seal 40, especially the interior of the cuff 42. When a fluid-based sterilization technique may be used for step 78, such a technique should not be used for the interior of the seal 40 because, as explained above, it may result in contamination of the swellable material 44. Instead, in some embodiments, a different non-fluid-contact sterilization technique may be used, for example, using radiation sterilization. Step 82 may be carried out at any suitable stage of the production process. In some embodiments, step 82 may be carried out as part of step 74. For example, the sub-assembly 50 may be sterilized so that it is provided at step 74 with the cuff 42 sterile (or at least having a sterile interior). Alternatively, step 82 may be carried out at any stage after step 76.

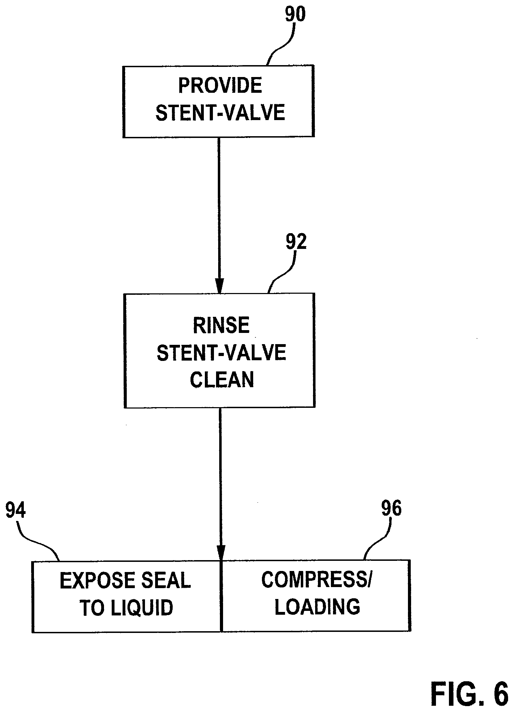

Referring to Fig. 6, a method of preparing the stent-valve 10 ready for implantation may comprise one or more of the following steps (any of which, and optionally all of which, may be carried out outside the body of the patient to be implanted) and only fall under the scope of claim 1 when carried out outside the body):
Step 90: providing the stent-valve 10 in a storage liquid, for example, as explained above;
Step 92: rinsing the stent-valve 10 to clean the storage liquid off the stent-valve 10. During step 92, the liquid-tight property of the cuff 42 prevents liquid contact with the swellable material 44. This permits thorough rinsing of the stent-valve 10 desirable to remove substantially all of the storage liquid.
Step 94: after step 92, exposing the swellable material 44 to permit contact with liquid; and
Step 96: after step 92, compressing the stent-valve 10 and/or loading the stent-valve 10 into a delivery apparatus 98 (Fig. 8).
Steps 94 and 96 may be carried out in either order or at least partly at the same time as each other.

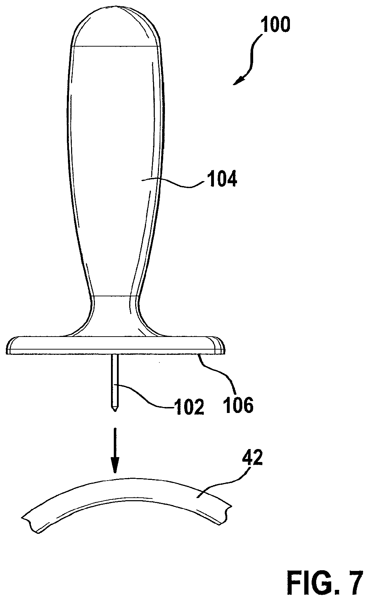

In some embodiments, step 96 may comprise the step of piercing the cuff 42 using a piercing tool 100, to penetrate the cuff material and create one or more liquid-admitting punctures in the cuff 42. Piercing the cuff 42 may leave the material of the cuff 42 in place. For example, if used, a diffusion barrier layer may remain in place on the stent-valve 10, even after implantation. The punctures created in the cuff 42 may pass through the diffusion barrier layer. An example piercing tool 100 is illustrated in Fig. 7. The piercing tool 100 may comprise at least one sharp pin 102 (or other sharp projection), and a handle portion 104 for enabling manual manipulation of the tool. The pin 102 may be dimensioned such that it can safely penetrate the cuff 42 without reaching through to the interior of the stent 12, and valve 14. Damage to the valve 14 can be prevented. In some embodiments, a face or flange 106 of the handle portion 104 may act as an abutment that bears against the cuff 42 surface to limit the depth of penetration, or another form of "stop" may be provided.In other embodiments, the piercing tool may comprise a roller having a surface on which is formed at least one sharp pin (preferably plural pins). In use, the roller is rolled on the surface to be pierced, and the punctures are created as the roller rolls against that surface.

Optionally, the step of piercing the cuff 42 may include piercing the cuff 42 at one or more positions that are clear of the location of the swellable material 44 within the cuff. Piercing the cuff 42 away from the swellable material 44 may avoid risk of physical damage to a swellable material component. In some embodiments, the cuff 42 may be transparent, or translucent, and the swellable material 44 may have a color (e.g. a distinctive color) to enable the location of the swellable material inside the cuff 42 to be identified. This can help the medical practitioner if it is desired to pierce the cuff 42 at positions clear of the location of the swellable material 44.

Additionally or alternatively, whether or not the cuff 42 is to be pierced at positions clear of the swellable material 44, the cuff 42 may comprise indicia to indicate suitable positions on the cuff 42 at which to pierce/penetrate the cuff material, to create the liquid-admitting punctures.

Step 94 is not limited only to piercing. A further example may be to remove a removable portion (e.g. liquid-tight cover) of the cuff described above, in order to expose the swellable material to contact by liquid.

Generally, the ability to complete the exposure step 94 prior to introduction into the patient's body can avoid any need to rely on an exposure mechanism that is activated as part of the implantation procedure once inside the body, for example, the pressure responsive rupturing capsules described in the aforementioned US-A-2007/0060998 and WO-A-2010/083558. This can reduce the risk of complication should, in some cases, such an exposure mechanism malfunction and fail to operate correctly at the time of implantation and once already in the body, where the possibility of further intervention may already be limited.

In some embodiments, step 96 may comprise using a compressing tool (such as one or more funnel shaped tubes, not shown) through which the stent-valve 10 is advanced in order to compress the stent-valve 10 to its compressed configuration. The stent valve 10 may be coupled to, and/or loaded within a constraining sheath 106 of, the delivery catheter 98. The constraining sheath 106 may constrain the stent-valve 10 in the compressed configuration suitable for introduction into the patient via minimally invasive surgery or a percutaneous procedure.

In some embodiments, step 96 may be carried out at least partly while contacting the stent-valve 10 with liquid, for example, at least partly immersing the stent-valve in liquid. The liquid may be water or saline. The liquid may be cold, for example, at a temperature less than room temperature (for example, cold water or cold saline). For example, carrying out the compressing step in cold liquid may make the stent 12 more supple and easier to compress. Additionally or alternatively, the containment sheath 106 may be flushed or at least partly filled with liquid to purge air from the containment sheath 106, prior to introduction into a patient's body.

In some embodiments, especially where step 96 is carried out at least partly while contacting the stent-valve 10 with liquid, it may be decided to carry out step 94 after the stent-valve 10 (or at least a portion of the stent-valve 10 carrying the seal 40) is constrained in a compressed condition by the constraining sheath 106. Such a technique can (i) permit at least partial exposure of the swellable material 44 to liquid to at least partly wet or hydrate the swellable material 44 prior to introduction into the patient's body, and (ii) prevent the seal 40 from swelling prematurely, even though the swellable material 44 is exposed to liquid.

Alternatively, step 94 may be carried out before (and/or during) step 96. The step of compressing the stent-valve may similarly be effective to constrain swelling and/or expansion of the seal 40 due to contact with liquid during step 94. In some embodiments, the stent-valve may be compressed progressively (for example, progressively from one end), and the compressed portion may pass into the constraining sheath of the delivery catheter generally straight after that portion has been compressed. Such a technique may be equally effective in restraining the seal against swelling, even though the swellable material is exposed to loquid and becomes at least partly wetted or hydrated.

Howsoever implemented, wetting or hydrating the swellable material 44, at least partly, prior to introduction into the body may in some cases be beneficial to enable more efficient swelling of the material 44, and therefore of the seal 40 and/or cuff 42, when the stent-valve 10 is implanted. It can avoid the need for the seal 40 to have to become wetted or to hydrate only on implantation. For example, speed of wetting and/or hydration and/or swelling may in some cases be a consideration if the liquid-admitting apertures (e.g. punctures) in the cuff 42 are relatively small and/or if a relatively "slow" wetting and/or hydrating and/or swelling material 44 is used within the cuff 42.

Additionally or alternatively, exposing the swellable material 44 only relatively late in the preparation procedure may combine (i) the advantage of being able to perform the exposure step 94 outside the patient's body (to avoid having to rely, as mentioned above, on an exposure mechanism that is activated as part of the implantation procedure once in the body), while (ii) limiting the amount of time during which the swellable material (44) is exposed to liquid prior to the implantation. Exposure during an excessive period of time might, in some cases and depending on the materials used, be counterproductive to the use as a dynamically swelling seal. In some embodiments, the swellable material 44 might be exposed to liquid outside the patient's body, for a time duration of: optionally not more than about 1 hour; optionally not more than about 30 minutes; optionally not more than about 20 minutes; optionally not more than about 15 minutes; optionally not more than about 10 minutes; optionally not more than about 9 minutes; optionally not more than about 8 minutes; optionally not more than about 7 minutes; optionally not more than about 6 minutes; optionally not more than about 5 minutes; optionally not more than about 4 minutes; optionally not more than about 3 minutes; optionally not more than about 2 minutes; optionally not more than about 1 minute.

Fig. 8 illustrates a portion of a delivery catheter 98, including a containment region 108 for the stent-valve 10 (indicated schematically in its compressed configuration by broken lines), and a constraining sheath (also referred to as a containment sheath) 106. The delivery catheter 98 is illustrated in a condition optionally outside the patient's body, but in which the stent-valve 10 is loaded, and the delivery catheter 98 may be ready for introduction into the patient's body. The constraining sheath 106 may be translatable between a closed condition (as shown) in which the sheath 106 substantially constrains the stent-valve 10 in its compressed configuration (or at least surrounds the seal 40), ready for introduction into the patient's body and delivery to the implantation site, and an open position (not shown) in which the sheath is translated in a direction (e.g. as illustrated by arrow 110 towards a handle portion 114, but optionally in the opposite direction away from the handle portion 114) to expose the stent-valve 10 for expansion to the operative configuration for implantation. The delivery catheter 98 may further comprise a flushing port 112 (which may optionally be at the handle portion 114 or handle-end of the delivery catheter). The flushing port 112 permits introduction of a liquid 116 (e.g. saline) for filling at least the containment region 108, and for purging trapped air from the containment region 108. The stent-valve 10 is immersed in the liquid 116 inside the containment sheath 106.

The sheath 106 may comprise a plurality of guide apertures 118 which, in the closed condition of the sheath 106, align with, or overlap or otherwise become in register with, the cuff 42 and/or seal 40. The guide apertures 112 are intended to permit insertion of the pin 102 of the piercing tool 100, in order to create liquid-admitting punctures in the cuff, as described earlier above. The liquid-admitting punctures may be formed before, or after, or during, the introduction of liquid 116 into the containment region 108. The punctures may cause the liquid 116 to come into contact with the swellable material 44 of the seal 40. However, the constraining sheath 106 can prevent substantial expansion of the seal 40 until the moment of implantation.

Fig. 9 illustrates an alternative version of the delivery catheter 98 comprising plural sheaths 106a and 106b. The sheaths may meet substantially end to end (as shown), or they may be at least partially overlapping (not shown). In a similar manner to that described above, at least one of the sheaths 106a and 106b may comprise guide apertures intended to permit insertion of the pin 102 of the piercing tool 100 to penetrate and pierce the cuff 42 of the stent-valve 10. Alternatively (as shown), a small gap 118 at the interface between the two sheaths 106a and 106b may provide the guide aperture for insertion of the piercing tool.

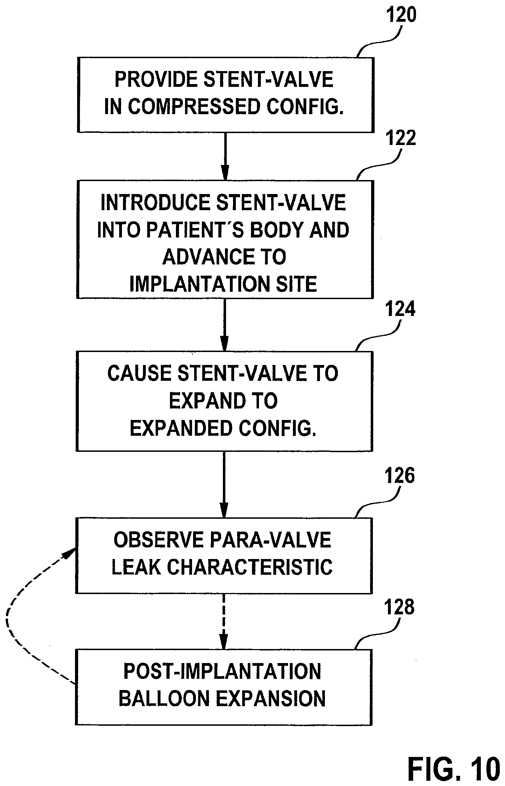

Referring to Fig. 10, a method of implanting the stent-valve 10 may comprise one or more of the following steps:
Step 120: providing the stent-valve 10 in its compressed configuration ready for introduction into a patient's body. Optionally this step may include the preparation steps of Fig. 6 and/or apparatus of any of Figs. 7 to 9;
Step 122: introducing the stent-valve 10 in its compressed configuration into the patient's body, and advancing the stent-valve to a desired implantation site. By way of example, if the cuff 42 may include a diffusion barrier layer 62, then step 122 may optionally include introducing the stent-valve 10 with the diffusion barrier layer 62 still in place on the cuff 42. Optionally, the cuff 42 may have been pierced at once or more positions to create liquid-admitting punctures in the cuff 42 that pass through the diffusion barrier layer 62.Alternatively, if the diffusion barrier layer 62 is provided only on a removable portion of the cuff, the diffusion barrier layer may be absent once the removable portion has been removed to expose the swellable material to liquid.
Step 124: causing the stent-valve 10 to expand at the implantation site, from the compressed configuration to the operative configuration. If the stent 12 is of a self-expanding type, the expansion may be caused by removing a constraining sheath (e.g. sheath 106), in order to allow the stent 12 to self-expand towards the operative configuration. Additionally or alternatively, if the stent 12 is of a type in which manipulation of the stent-valve 10 is used to cause the stent-valve 10 to adopt its operative configuration, step 124 may include causing such manipulation, for example, by inflating an expansion balloon and/or foreshortening the stent 12 to a foreshortened state.
Step 126: observing one or more characteristics of the operative stent-valve. For example, one such characteristic may be the extent of para-valve leakage of blood. Such a characteristic may be observed using any suitable technique, for example, Doppler-effect ultrasound. Additionally or alternatively, the implantation position and/or the extent to which the stent-valve has expanded, and/or the pressure gradient through the valve, may be observed.
Step 128: in dependence of the result of the observation in step 126, performing post-implantation balloon expansion of the stent-valve 10. For example, if the observation of step 126 indicates that a para-valve leakage condition is not acceptable, and/or that the stent has not expanded as much as desired, and/or that the pressure gradient is undesirably high, a balloon catheter may be inserted into the interior of stent 12, and expanded to improve the seating/expansion of the stent 12 within the native anatomy at the implantation site. If the observation at step 126 indicates that a para-valve leakage condition and/or other condition is acceptable (for example, there is no substantial leakage), then step 128 may be skipped.
Step 128 may be performed after a time interval sufficient to permit swelling of the seal 40 to adapt to the native anatomy. For example, the time interval may be at least about 30 seconds, optionally at least about 40 seconds, optionally at least about 50 seconds, optionally at least about 1 minute, optionally at least about 75 seconds, optionally at least about 90 seconds, optionally at least about 105 seconds, optionally at least about 2 minutes, optionally at least about two-and-a-half minutes, optionally at least about 3 minutes, optionally at least about three- and-a-half minutes, optionally at least about 4 minutes, optionally at least about four-and-a-half minutes, optionally at least about 5 minutes. Additionally or alternatively, the time interval may optionally be not substantially more than about 10 minutes, optionally not substantially more than about 9 minutes, optionally not substantially more than about 8 minutes, optionally not substantially more than about 7 minutes, optionally not substantially more than about 6 minutes, optionally not substantially more than about 5 minutes, optionally not substantially more than about 4 minutes, optionally not substantially more than about 3 minutes, optionally not substantially more than about 2 minutes, optionally not substantially more than about 1 minute.

It may not be intuitive to consider carrying out post-implantation balloon-expansion of a stent-valve that includes a swellable seal 40, because it might ordinarily be expected that the seal 40 will be able to seal against the anatomy automatically. However, steps 126 and 128 may permit the medical practitioner to determine, at least prior to completion of the medical procedure and while the patient is still in a condition ready for intervention, the efficacy of the seal 40 in sealing between the stent-valve 10 and the surrounding local anatomical tissue. If the seal 40 is determined not to be sufficiently effective, then step 128 may be used to increase the seating of the stent-valve 10 within the local anatomy, and the associated sealing effect of the seal 40. Steps 126 and 128 may be performed once, or repeated two or more times, as desired, for example, until para-valve leakage is reduced to an acceptable condition.

As explained earlier above, the seal 40 may be configured to be able to withstand a post-implantation balloon-expansion procedure, without risk of bursting.

Although the foregoing description has described the embodiments in terms of a stent-valve 10, it will be appreciated that many of the same techniques may be applied to other stented prostheses.

It is emphasized that the foregoing description of preferred embodiments does not limit the scope of the invention, and that many alternatives, modifications, and improvements may be made within the scope and/or principles of the invention as defined in the claims.

## Claims

1. A method comprising
providing a stent-valve delivery system outside the body of a patient, the stent-valve delivery system comprising:
a delivery catheter (98) loaded with a stent-valve (10), the stent-valve (10) comprising a seal (40) comprising swellable material (44) that swells when contacted by liquid (116), and the delivery catheter (98) comprising a containment sheath (106) encompassing at least a portion of the stent-valve (10) at which the seal is located,
**characterized by**
the containment sheath (106) at least partly filled with liquid (116) outside the body of a patient, the swellable material (44) exposed at least partly to the liquid (116), the containment sheath (106) obstructing expansion and/or outward swelling of the seal (40).

2. A stent-valve delivery system outside the body of a patient comprising:
a delivery catheter (98) loaded with a stent-valve (10), the stent-valve (10) comprising a seal (40) comprising swellable material (44) that swells when contacted by liquid (116),
**characterized by**
the swellable material (44) being in an at least partly hydrated condition by contact with liquid (116), and the delivery catheter (98) comprising a containment sheath (106) encompassing at least a portion of the stent-valve (10) at which the seal (40) is located, the containment sheath obstructing outward swelling of the at least partly hydrated swellable material (44).

3. The method according to claim 1 or the system according to claim 2 wherein the liquid is or comprises saline.

4. The method of claim 1 or 3, or the system of claim 2 or 3, wherein
the delivery catheter (98) further comprises
a port (112) for introduction of the liquid (116) into the containment sheath (106).

5. The method according to claim 1, 3 or 4, or a system according to claim 2, 3 or 4, wherein the swellable material (44) is or comprises a hydrogel.

6. The method according to claim 1, 3, 4 or 5, or a system according to claim 2, 3, 4, or 5, wherein the seal (40) further comprises a hollow cuff (42).

7. The method of claim 6 or the system of claim 6, wherein the cuff (42) extends in a generally circumferential direction around a stent (12) of the stent-valve (10).

8. The method of claim 6 or 7 or the system of claim 6 or 7, wherein the swellable material (44) is arranged within the cuff (42).

9. The method of claim 6, 7 or 8 of the system of claim 6, 7 or 8, further comprising a skirt (30, 32) secured to the hollow cuff (42).

10. The method of claim 9 or the system of claim 9, wherein the skirt (30, 32) is at least one selected from: an outer skirt (32) arranged generally outside a stent (12) of the stent-valve (10); an inner skirt (30) arranged generally within a stent (12) of the stent-valve (10).

11. The method of claim 6, 7, 8, 9 or 10 or the system of claim 6, 7, 8, 9, or 10, wherein the hollow cuff (42) comprises a film (64) made of liquid-impermeable material, the cuff (42) having one or more liquid admitting punctures made therein, prior to introduction of the stent-valve into the body of a patient, for admitting liquid (112) into the seal (40).

12. The method according claim 1 or any claim dependent thereon or the system according to claim 2 or any claim dependent thereon, wherein the seal (40) is arranged between the respective extremities of first and second crowns (16, 18) of a stent component (12) of the stent-valve (10).

13. The method according claim 1 or any claim dependent thereon or the system according to claim 2 or any claim dependent thereon, wherein the containment sheath (106) is slidable to a position in which the sheath (106) no longer surrounds the stent-valve (10), to permit deployment of the stent-valve (10) from the delivery catheter (98).

## Patentansprüche

1. Verfahren, umfassend:
Bereitstellen eines Stentklappenzuführsystems außerhalb des Körpers eines Patienten, wobei das Stentklappenzuführsystem Folgendes umfasst:
einen mit einer Stentklappe (10) beladenen Zuführkatheter (98), wobei die Stentklappe (10) eine Dichtung (40) umfasst, die quellfähiges Material (44) umfasst, das bei Kontakt mit Flüssigkeit (116) quillt, und der Zuführkatheter (98) eine Umschließungshülle (106) umfasst, die mindestens einen Abschnitt der Stentklappe (10), an dem die Dichtung angeordnet ist, umschließt,
**dadurch gekennzeichnet, dass**
die Umschließungshülle (106) außerhalb des Körpers eines Patienten mindestens teilweise mit Flüssigkeit (116) gefüllt wird und das quellfähige Material (44) mindestens teilweise der Flüssigkeit (116) ausgesetzt wird, wobei die Umschließungshülle (106) die Dichtung (40) an der Expansion und/oder daran hindert, nach außen zu quellen.

2. Stentklappezuführsystem außerhalb des Körpers eines Patienten, umfassend:
einen mit einer Stentklappe (10) beladenen Zuführkatheter (98), wobei die Stentklappe (10) eine Dichtung (40) umfasst, die quellfähiges Material (44) umfasst, das bei Kontakt mit Flüssigkeit (116) quillt,
**dadurch gekennzeichnet, dass**
das quellfähige Material (44) durch Kontakt mit Flüssigkeit (116) in einem mindestens teilweise hydratisierten Zustand ist und der Zuführkatheter (98) eine Umschließungshülle (106) umfasst, die mindestens einen Abschnitt der Stentklappe (10), an dem die Dichtung (40) angeordnet ist, umschließt, wobei die Umschließungshülle das mindestens teilweise hydratisierte, quellfähige Material (44) daran hindert, nach außen zu quellen.

3. Verfahren nach Anspruch 1 oder System nach Anspruch 2, wobei die Flüssigkeit Kochsalzlösung ist oder umfasst.

4. Verfahren nach Anspruch 1 oder 3 oder System nach Anspruch 2 oder 3, wobei der Zuführkatheter (98) ferner einen Port (112) zur Einführung der Flüssigkeit (116) in die Umschließungshülle (106) umfasst.

5. Verfahren nach Anspruch 1, 3 oder 4 oder System nach Anspruch 2, 3 oder 4, wobei das quellfähige Material (44) ein Hydrogel ist oder umfasst.

6. Verfahren nach Anspruch 1, 3, 4 oder 5 oder System nach Anspruch 2, 3, 4 oder 5, wobei die Dichtung (40) ferner einen hohlen Cuff (42) umfasst.

7. Verfahren nach Anspruch 6 oder System nach Anspruch 6, wobei sich der Cuff (42) in einer allgemeinen Umfangsrichtung um einen Stent (12) der Stentklappe (10) herum erstreckt.

8. Verfahren nach Anspruch 6 oder 7 oder System nach Anspruch 6 oder 7, wobei das quellfähige Material (44) im Cuff (42) angeordnet ist.

9. Verfahren nach Anspruch 6, 7 oder 8 oder System nach Anspruch 6, 7 oder 8, ferner umfassend eine Manschette (30, 32), die am hohlen Cuff (42) befestigt ist.

10. Verfahren nach Anspruch 9 oder System nach Anspruch 9, wobei die Manschette (30, 32) mindestens eine ist, die aus Folgendem ausgewählt ist: einer äußeren Manschette (32), die allgemein außerhalb eines Stents (12) der Stentklappe (10) angeordnet ist, einer inneren Manschette (30), die allgemein innerhalb eines Stents (12) der Stentklappe (10) angeordnet ist.

11. Verfahren nach Anspruch 6, 7, 8, 9 oder 10 oder System nach Anspruch 6, 7, 8, 9 oder 10, wobei der hohle Cuff (42) eine aus flüssigkeitsdurchlässigem Material hergestellte Folie (64) umfasst, wobei vor der Einführung der Stentklappe in den Körper eines Patienten ein oder mehrere Einstiche, die Flüssigkeit hereinlassen, in den Cuff (42) vorgenommen werden, um Flüssigkeit (112) in die Dichtung (40) hereinzulassen.

12. Verfahren nach Anspruch 1 oder einem davon abhängigen Anspruch oder System nach Anspruch 2 oder einem davon abhängigen Anspruch, wobei die Dichtung (40) zwischen den jeweiligen Enden der ersten und der zweiten Krone (16, 18) einer Stentkomponente (12) der Stentklappe (10) angeordnet ist.

13. Verfahren nach Anspruch 1 oder einem davon abhängigen Anspruch oder System nach Anspruch 2 oder einem davon abhängigen Anspruch, wobei die Umschließungshülle (106) in eine Position verschiebbar ist, in der die Hülle (106) die Stentklappe (10) nicht mehr umgibt, um ein Abgeben der Stentklappe (10) vom Zuführkatheter (98) zu gestatten.

## Revendications

1. Procédé comprenant
la fourniture d'un système de largage d'endoprothèse-valve à l'extérieur du corps d'un patient, le système de largage d'endoprothèse-valve comprenant :
un cathéter de largage (98) chargé d'une endoprothèse-valve (10), l'endoprothèse-valve (10) comprenant un joint (40) comprenant un matériau gonflable (44) qui gonfle lorsqu'il est mis en contact avec un liquide (116), et le cathéter de largage (98) comprenant une gaine de retenue (106) englobant au moins une portion de l'endoprothèse-valve (10) au niveau de laquelle le joint est situé,
**caractérisé par**
la gaine de retenue (106) remplie au moins en partie de liquide (116) à l'extérieur du corps d'un patient, le matériau gonflable (44) exposé au moins en partie au liquide (116), la gaine de retenue (106) obstruant une expansion et/ou un gonflement vers l'extérieur du joint (40).

2. Système de largage d'endoprothèse-valve à l'extérieur du corps d'un patient, comprenant :
un cathéter de largage (98) chargé d'une endoprothèse-valve (10), l'endoprothèse-valve (10) comprenant un joint (40) comprenant un matériau gonflable (44) qui gonfle lorsqu'il est mis en contact avec un liquide (116),
**caractérisé par**
le matériau gonflable (44) étant dans un état au moins en partie hydraté par un contact avec un liquide (116), et le cathéter de largage (98) comprenant une gaine de retenue (106) englobant au moins une portion de l'endoprothèse-valve (10) au niveau de laquelle le joint (40) est situé, la gaine de retenue obstruant un gonflement vers l'extérieur du matériau gonflable (44) au moins en partie hydraté.

3. Procédé selon la revendication 1 ou système selon la revendication 2, dans lequel le liquide est ou comprend une solution saline.

4. Procédé selon la revendication 1 ou 3, ou système selon la revendication 2 ou 3, dans lequel le cathéter de largage (98) comprend en outre
un orifice (112) pour une introduction du liquide (116) dans la gaine de retenue (106).

5. Procédé selon la revendication 1, 3 ou 4, ou système selon la revendication 2, 3 ou 4, dans lequel le matériau gonflable (44) est ou comprend un hydrogel.

6. Procédé selon la revendication 1, 3, 4 ou 5, ou système selon la revendication 2, 3, 4 ou 5, dans lequel le joint (40) comprend en outre un manchon creux (42).

7. Procédé selon la revendication 6 ou système selon la revendication 6, dans lequel le manchon (42) s'étend dans une direction généralement circonférentielle autour d'une endoprothèse (12) de l'endoprothèse-valve (10).

8. Procédé selon la revendication 6 ou 7 ou système selon la revendication 6 ou 7, dans lequel le matériau gonflable (44) est agencé au sein du manchon (42).

9. Procédé selon la revendication 6, 7 ou 8 ou système selon la revendication 6, 7 ou 8, comprenant en outre une jupe (30, 32) arrimée au manchon creux (42).

10. Procédé selon la revendication 9 ou système selon la revendication 9, dans lequel la jupe (30, 32) est au moins l'une choisie parmi : une jupe externe (32) agencée généralement à l'extérieur d'une endoprothèse (12) de l'endoprothèse-valve (10) ; une jupe interne (30) agencée généralement au sein d'une endoprothèse (12) de l'endoprothèse-valve (10).

11. Procédé selon la revendication 6, 7, 8, 9 ou 10 ou système selon la revendication 6, 7, 8, 9 ou 10, dans lequel le manchon creux (42) comprend un film (64) réalisé en un matériau imperméable aux liquides, le manchon (42) ayant une ou plusieurs perforations d'admission de liquide réalisées à l'intérieur, avant introduction de l'endoprothèse-valve dans le corps d'un patient, pour admettre du liquide (112) dans le joint (40).

12. Procédé selon la revendication 1 ou l'une quelconque des revendications dépendant de celle-ci ou système selon la revendication 2 ou l'une quelconque des revendications dépendant de celle-ci, dans lequel le joint (40) est agencé entre les extrémités respectives de première et seconde couronnes (16, 18) d'un composant endoprothèse (12) de l'endoprothèse-valve (10).

13. Procédé selon la revendication 1 ou l'une quelconque des revendications dépendant de celle-ci ou système selon la revendication 2 ou l'une quelconque des revendications dépendant de celle-ci, dans lequel la gaine de retenue (106) est coulissante jusqu'à une position dans laquelle la gaine (106) n'entoure plus l'endoprothèse-valve (10), pour permettre un déploiement de l'endoprothèse-valve (10) depuis le cathéter de largage (98).
